# FASCICULE DE BREVET EUROPEEN

(11) **EP 1 475 075 B1**
(45) Date de publication et mention de la délivrance du brevet: **22.07.2009**
(21) Numéro de dépôt: 04291136.2
(22) Date de dépôt: 04.05.2004
(51) Int. Cl.: A61Q 5/04, A61K 8/81

(54) **Défrisage alcalin en présence d'un polymère hydrosoluble de haut poids moléculaire**
Alkalisches Glätten der Haare in Gegenwart eines wasserlöslichen Polymers mit hohem Molekulargewicht
Alcaline straightening of the hair in the presence of a hydrosoluble polymer having a high molecular weight

(30) Priorité: 05.05.2003 FR 0305451
(43) Date de publication de la demande: 10.11.2004
(73) Titulaire: L'ORÉAL, 75008 Paris (FR)
(72) Inventeur: Samain, Henri, 91570 Bievres (FR); Livoreil, Aude, 75006 Paris (FR)
(74) Mandataire: Dossmann, Gérard

(56) Documents cités:
- WO-A-91/04007
- US-A- 5 639 449
- US-A- 5 641 478

## Description

La présente invention concerne l'utilisation d'une dispersion d'un polymère hydrosoluble de haut poids moléculaire pour protéger les fibres kératiniques contre la dégradation chimique lors du défrisage alcalin des cheveux, un kit multi-compartiments pour le défrisage alcalin contenant une dispersion ou une solution d'un tel polymère, une composition de défrisage prête à l'emploi et un procédé de défrisage alcalin utilisant un tel kit ou une telle composition prête à l'emploi.

Les procédés de défrisage ou de décrêpage connus reposent essentiellement sur deux types de traitements chimiques :
- le traitement des fibres kératiniques avec un agent réducteur provoquant la coupure des ponts disulfure (S-S) de la kératine, suivi d'un traitement oxydant appliqué sur les cheveux soumis à une contrainte mécanique, et
- le traitement des fibres kératiniques soumises à une contrainte mécanique avec un agent fortement alcalin.

Ces deux types de traitements permettent de détendre efficacement les boucles, mais présentent l'inconvénient d'être très agressifs et de modifier la structure chimique de la kératine.

La répétition de ces traitements se traduit généralement par de mauvaises propriétés cosmétiques des cheveux telles qu'un démêlage difficile, un toucher désagréable ou des cheveux rêches et ternes, mais surtout par une dégradation des fibres kératiniques.

Cette dégradation des fibres est particulièrement indésirable car elle détériore de manière irréversible les propriétés physico-chimiques des cheveux. Ceux-ci deviennent plus poreux et par conséquent plus difficile à sécher. Ils présentent une plus grande sensibilité aux divers autres traitements capillaires tels qu'une coloration, et voient leurs propriétés mécaniques et leurs propriétés de surface modifiées défavorablement, ce qui se traduit par exemple par une diminution de la résistance à la rupture en traction ou une augmentation du coefficient de frottement.

La demanderesse a découvert avec surprise qu'il était possible de réduire significativement la dégradation chimique des fibres kératiniques résultant du traitement alcalin de défrisage, en utilisant l'agent de défrisage alcalin en combinaison avec au moins un polymère choisi parmi les polymères de haut poids moléculaire, obtenus par polymérisation radicalaire hétérogène de monomères hydrosolubles avec précipitation du polymère formé dans le milieu réactionnel.

Ce qui est particulièrement surprenant est le fait que l'effet de protection des fibres kératiniques est observé uniquement pour le procédé de défrisage alcalin mais non pas pour un procédé de défrisage par réduction/oxydation de la kératine.

L'effet de protection des fibres kératiniques est obtenu non seulement en cas d'utilisation conjointe (application simultanée) de l'agent de défrisage alcalin et du polymère de haut poids moléculaire, mais également lorsqu'on utilise un procédé de défrisage en deux temps comprenant d'abord le traitement des fibres kératiniques avec le polymère protecteur puis l'étape de défrisage alcalin, ces deux étapes pouvant être séparées par une étape de rinçage intermédiaire. Un effet protecteur moindre est obtenu en cas d'application du polymère de haut poids moléculaire après l'étape de défrisage alcalin.

Comme expliqué en détail ci-après, les polymères utilisés dans la présente invention sont obtenus par polymérisation radicalaire hétérogène d'un mélange de monomères solubles dans l'eau. Le milieu de polymérisation est une solution aqueuse saline ayant une force ionique relativement élevée, choisie de manière à ce que le polymère, soluble dans l'eau pure, précipite au fur et à mesure de sa formation de manière à former une dispersion de fines particules de polymère dans une phase aqueuse saline continue. Cette dispersion présente l'avantage de présenter à la fois une concentration assez importante en polymère de haut poids moléculaire et une viscosité relativement modérée permettant un mélange rapide et facile de cette dispersion avec l'agent de défrisage alcalin. Cette dernière propriété permet l'utilisation des dispersions de polymère hydrosoluble de haut poids moléculaire dans des kits de défrisage où l'agent alcalin et le polymère hydrosoluble sont conditionnés dans des compartiments séparés et sont mélangés immédiatement avant application sur les cheveux.

La présente invention a par conséquent pour objet un agent de défrisage alcalin multi-composants comportant,
- en tant que premier composant, (a) une composition aqueuse alcaline de défrisage, et,
- en tant que deuxième composant, (b1) une composition contenant une dispersion de particules d'un polymère hydrosoluble, de préférence un polyélectrolyte, ayant une masse moléculaire moyenne en poids supérieure à 10⁶ dans une solution aqueuse saline, obtenu par polymérisation radicalaire hétérogène de monomères hydrosolubles avec précipitation du polymère formé, les deux composants (a) et (b1) étant destinés à être appliqués simultanément ou successivement sur les fibres kératiniques à défriser.

Le polymère hydrosoluble ayant une masse moléculaire moyenne en poids supérieure à 10⁶ peut également être, non pas sous forme dispersée, mais sous forme dissoute dans un milieu aqueux physiologiquement acceptable. La présente invention a par conséquent également pour objet un agent de défrisage tel que décrit ci-dessus comportant, à la place du composant (b1), un composant (b2) sous forme d'une composition contenant une solution aqueuse d'un polymère hydrosoluble ayant une masse moléculaire moyenne en poids supérieure à 10⁶, obtenu par polymérisation radicalaire hétérogène de monomères hydrosolubles avec précipitation du polymère formé.

L'agent de défrisage alcalin multi-composants de l'invention se présente de préférence sous forme d'un kit multi-compartiments pour le défrisage alcalin des fibres kératiniques, avec au moins un premier compartiment contenant le premier composant (a) et au moins un deuxième compartiment contenant le deuxième composant (b1) ou (b2).

Comme indiqué ci-dessus, la synthèse des polymères hydrosolubles de haut poids moléculaire, utilisés dans la présente invention pour la protection des fibres kératiniques contre la dégradation alcaline, se fait par polymérisation radicalaire hétérogène de monomères hydrosolubles comportant au moins une insaturation éthylénique. La polymérisation se fait dans une solution aqueuse d'un électrolyte minéral (sel) ayant une force ionique suffisante pour provoquer la précipitation du polymère formé dès que celui-ci atteint une certaine masse moléculaire. Cette technique de polymérisation permet ainsi, grâce au phénomène bien connu de relargage par des sels *(salting-out),* la préparation de dispersions aqueuses salines de particules de polymères hydrosolubles. Les polymères ainsi synthétisés se distinguent par une masse moléculaire moyenne en poids élevée, généralement supérieure à 10⁶.

La technique de polymérisation radicalaire hétérogène en milieu aqueux avec précipitation du polymère formé est décrite par exemple dans le brevet US 4 929 655, dans la demande de brevet EP 0 943 628 ou encore dans la demande internationale WO 02/34796.

Pour garantir la stabilité des dispersions de particules de polymère pendant la synthèse et au cours du stockage, il est généralement indispensable de réaliser la polymérisation en présence d'un agent dispersant. Cet agent dispersant est de préférence un polyélectrolyte qui, contrairement au polymère de haut poids moléculaire utilisé dans l'invention, est soluble dans le milieu aqueux de polymérisation de force ionique élevée.

Ce polyélectrolyte dispersant a de préférence une charge identique à celle du polymère synthétisé, autrement dit pour la synthèse de polyélectrolytes cationiques, on utilise généralement un polyélectrolyte dispersant cationique.

On peut citer à titre d'agents dispersants préférés, les polyélectrolytes cationiques obtenus par polymérisation de 50 à 100 % en moles d'au moins un monomère cationique choisi parmi les sels, de préférence les chlorhydrates ou sulfates, de (méth)acrylate de diméthylaminoéthyle, de N-diméthylaminopropyl(méth)acrylamide ou de di(méth)allylamine, le chlorure de (méth)acryloyloxyéthyltriméthylammonium, le chlorure de (méth)acrylamido-propyltriméthylammonium et le chlorure de diméthyldiallylammonium, et de 50 à 0 % en moles d'acrylamide. On peut également utiliser une polyamine telle qu'une polyalkylèneamine.

L'agent dispersant est utilisé de préférence à raison de 1 à 10 % en poids, rapporté au poids total des monomères à polymériser.

La solution aqueuse saline qui sert de milieu de synthèse et de dispersion au polymère hydrosoluble de haut poids moléculaire est une solution d'un ou de plusieurs sels minéraux choisis de préférence parmi les sels anioniques divalents. On peut citer en tant que sels anioniques divalents par exemple le sulfate d'ammonium, l'hydrogénosulfate d'ammonium, le sulfate de sodium, l'hydrogénosulfate de sodium, le sulfate de magnésium, l'hydrogénosulfate de magnésium, le sulfate d'aluminium et l'hydrogénosulfate d'aluminium. Le sulfate d'ammonium et le sulfate de sodium sont particulièrement préférés.

La concentration de ce(s) sel(s) doit de préférence être suffisante pour induire la précipitation du polymère hydrosoluble formé dans le milieu de polymérisation. Pour obtenir une telle précipitation, la concentration en sel est de préférence au moins égale à 15 % en poids et peut aller jusqu'à la concentration de saturation de chaque sel. La solution aqueuse saline peut contenir en outre des sels monovalents tels que le chlorure de sodium et le chlorure d'ammonium.

La polymérisation radicalaire hétérogène en milieu aqueux telle que décrite ci-dessus s'accompagne le plus souvent d'une augmentation importante de la viscosité du milieu réactionnel, qui se traduit par des difficultés d'agitation, un défaut d'homogénéité du milieu réactionnel et un élargissement de la granulométrie des particules de polymère formé. Pour empêcher une telle augmentation de la viscosité, il a été proposé, dans la demande de brevet européen EP 0 943 628, d'ajouter au milieu de polymérisation au moins un agent empêchant l'augmentation de la viscosité du milieu réactionnel en cours de polymérisation.

Les polymères hydrosolubles de haut poids moléculaire utilisés dans la présente invention sont de préférence préparés en présence d'un tel agent empêchant l'augmentation de la viscosité.

Ces agents empêchant l'augmentation de la viscosité du milieu réactionnel sont choisis par exemple parmi
(A) les acides polycarboxyliques et leurs sels,
(B) les polyphénols,
(C) les composés cycliques contenant un groupe hydroxy et un groupe carboxy, ou leurs sels,
(D) l'acide gluconique ou ses sels,
(E) les produits réactionnels obtenus par réaction d'une méthoxyhydroquinone et/ou d'un monomère cationique (méth)acrylique avec un composé qui génère des radicaux, sous une atmosphère oxydante,
(F) les produits réactionnels obtenus par réaction d'un polymère cationique (méth)acrylique avec un composé qui génère des radicaux, sous une atmosphère oxydante,
(G) les produits réactionnels obtenus par réaction d'un polymère cationique (méth)acrylique avec un agent oxydant,
et leurs mélanges.

L'addition d'au moins un agent empêchant l'augmentation de la viscosité tel que décrit ci-dessus permet d'effectuer la polymérisation des monomères hydrosolubles décrits ci-dessus avec un agitateur à faible puissance tout en évitant la formation de particules grossières. Les agents empêchant l'augmentation de la viscosité sont de préférence solubles dans le milieu réactionnel aqueux.

Comme exemples de composés (A), on peut citer l'acide oxalique, l'acide adipique, l'acide tartrique, l'acide malique, l'acide phtalique et leurs sels.

Comme exemples de composés (B), on peut notamment citer le résorcinol et le pyrogallol.

Comme exemples de composés (C), on peut citer l'acide m-hydroxybenzoïque, l'acide p-hydroxybenzoïque, l'acide salicylique, l'acide gallique, l'acide tannique et les sels de ces acides.

Comme exemples de composés (D), on peut citer le gluconate de sodium, le gluconate de potassium, le gluconate d'ammonium et différents sels aminés de l'acide gluconique.

Comme exemples de composés (E), on peut citer ceux obtenus par réaction d'un composé qui génère des radicaux, sous un courant de gaz oxygéné, dans une solution contenant de la méthoxyhydroquinone et/ou un monomère cationique (méth)acrylique. Le composé qui génère des radicaux peut être un amorceur couramment utilisé pour la polymérisation radicalaire. On peut citer par exemple les amorceurs azoïques hydrosolubles tels que le chlorhydrate de 2,2'-azo-bis(2-amidinopropane) vendu par exemple sous la dénomination V-50 par la société Wako Chemical Industries, ou le chlorhydrate de 2,2'-azo-bis[2-(2-imidazoline-2-yl)propane] vendu par exemple sous la dénomination commerciale VA-044 par la société Wako Chemical Industries, ou un amorceur du groupe des oxydoréducteurs hydrosolubles tels que l'association persulfate d'ammonium/hydrogénosulfite de sodium.

On peut obtenir un agent empêchant l'augmentation de la viscosité (F) par réaction d'un amorceur radicalaire, sous atmosphère oxygénée, avec un agent dispersant selon l'invention. L'amorceur de polymérisation peut être un amorceur azoïque hydrosoluble ou un oxydoréducteur hydrosoluble tels que décrits ci-dessus.

Les composés (G) peuvent être obtenus sous la forme de polymères oxydés de faible masse moléculaire par oxydation d'un agent dispersant cationique selon l'invention obtenu par polymérisation d'un monomère cationique (méth)acrylique, utilisant en tant qu'agent oxydant du peroxyde d'hydrogène ou un halogène.

Comme monomères cationiques (méth)acryliques utilisés pour la préparation des agents empêchant l'augmentation de la viscosité (E), (F) et (G), on peut citer par exemple le chlorhydrate ou le sulfate de (méth)acrylate de diméthylaminoéthyle, le chlorure de (méth)acryloyloxyéthyltriméthylammonium, le chlorure de (méth)acryloyloxyéthyldiméthylbenzylammonium, le chlorhydrate ou le sulfate dérivé du N-diméthylaminopropyl(meth)-acrylamide, le chlorure de (méth)acrylamidopropyltriméthylammonium, le chlorure ou le sulfate de (méth)acrylate de diméthylaminohydroxypropyle, le chlorure de (méth)acryloyloxyhydroxypropyltriméthylammonium, le chlorure de (méth)acryloyloxyhydroxypropyldiméthylbenzylammonium.

Ces agents empêchant l'augmentation de la viscosité (A) à (G) peuvent être utilisés seuls ou en mélange, en une quantité comprise de préférence entre 10 ppm et 10 000 ppm par rapport au poids total de la solution réactionnelle.

Les monomères hydrosolubles polymérisés par polymérisation radicalaire hétérogène en vue de l'obtention des polymères hydrosolubles de haut poids moléculaire sont des monomères comportant au moins une double liaison éthylénique, par exemple une double liaison vinylique, acrylique ou allylique. Ils peuvent être cationiques, anioniques ou non-ioniques et être utilisés en mélange.

On peut citer à titre d'exemples de monomères anioniques hydrosolubles l'acide acrylique, l'acide méthacrylique, l'acide acrylamido-2-méthylpropanesulfonique et l'acide itaconique. Ces monomères anioniques sont au moins partiellement neutralisés sous forme d'un sel d'un métal alcalin, d'un métal alcalino-terreux, d'ammonium ou d'une amine organique telle que l'alcanolamine.

A titre de monomères non-ioniques hydrosolubles, on peut citer l'acrylamide, le méthacrylamide, le N-vinylformamide, le N-vinylacétonamide, l'acrylate d'hydroxypropyle et le méthacrylate d'hydroxypropyle.

Les monomères cationiques hydrosolubles sont de préférence choisis parmi les sels de di(alkyle en C₁₋₄)-diallylammonium et les composés de formule (I) 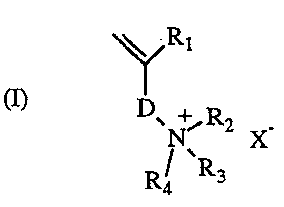 dans laquelle
R₁ représente un atome d'hydrogène ou un groupe méthyle,
R₂ et R₃, identiques ou différents, représentent chacun un atome d'hydrogène ou un groupe alkyle en C₁₋₄ linéaire ou ramifié,
R₄ représente un atome d'hydrogène, un groupe alkyle en C₁₋₄ linéaire ou ramifié ou un groupe aryle,
D représente le motif suivant 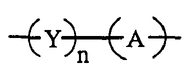 dans lequel Y représente une fonction amide (-CO-NH-), ester (-O-CO- ou - CO-O-), uréthane (-O-CO-NH-) ou urée (-NH-CO-NH-),
A représente un groupe alkyène en C₁₋₁₀ linéaire, ramifié ou cyclique, pouvant être substitué ou interrompu par un cycle aromatique ou hétéroaromatique divalent, ou pouvant être interrompu par un hétéroatome choisi parmi O, N, S et P, et pouvant comporter une fonction cétone, amide, ester, uréthane ou urée,
n vaut 0 ou 1, et
X⁻ représente un contre-ion anionique tel qu'un ion chlorure ou sulfate.

A titre d'exemples de monomères cationiques hydrosolubles, on peut citer le chlorhydrate ou le sulfate de (méth)acrylate de diméthylaminoéthyle, le chlorure de (méth)acryloyloxyéthyltriméthyl-ammonium, le chlorure de (méth)acryloyloxyéthyldiméthylbenzylammonium, le chlorhydrate ou sulfate de N-diméthylaminopropyl-(méth)acrylamide, le chlorure de (méth)acrylamidopropyltriméthylammonium, le chlorure de (méth)acrylamidopropyldiméthylbenzylammonium, le chlorhydrate ou sulfate de (méth)acrylate de diméthylaminohydroxypropyle, le chlorure de (méth)acryloyloxyhydroxypropyltriméthylammonium, le chlorure de (méth)acryloyloxyhydroxypropyldiméthylbenzylammonium et le chlorure de diméthyldiallylammonium.

Le polymère hydrosoluble de haut poids moléculaire est de préférence un polyélectrolyte, et en particulier un polyélectrolyte cationique ou amphotère, c'est-à-dire un polyélectrolyte polymérisé à partir d'au moins un monomère cationique de formule (I).

Dans un mode de réalisation préféré de l'agent de défrisage de la présente invention, le polymère hydrosoluble de haut poids moléculaire est obtenu par polymérisation radicalaire hétérogène d'un mélange de monomères constitué de 0 à 30 % en moles d'acide acrylique, de 0 à 95,5 % en moles d'acrylamide et de 0,5 à 10 % en moles d'au moins un monomère cationique de formule (I).

Selon un mode de réalisation particulièrement préféré, les polymères hydrosolubles sont obtenus par polymérisation d'un mélange de monomères constitué d'acide acrylique et d'un monomère cationique de formule (I), dans lequel le nombre de moles de monomère cationique de formule (I) est supérieur au nombre de moles d'acide acrylique.

Des polyélectrolytes hydrosolubles particulièrement préférés sont par exemple ceux polymérisés à partir de mélanges de monomères constitués respectivement
- de 10 % en moles de chlorure d'acryloyloxyéthyldiméthylbenzylammonium et de 90 % en moles d'acrylamide.
- de 30 % en moles de chlorure d'acryloyloxytriméthylammonium, de 50 % en moles de chlorure d'acryloyloxyéthyldiméthybenzylammonium et de 20 % en moles d'acrylamide.
- de 10 % en moles de chlorure d'acryloyloxyéthyltriméthylammonium et de 90 % en moles d'acrylamide.
- de 30 % en moles de chlorure de diallyldiméthylammonium et de 70 % en moles d'acrylamide.

Le polymère hydrosoluble de haut poids moléculaire peut également être un polyélectrolyte anionique polymérisé à partir d'un mélange de monomères anioniques et de monomères non-ioniques. On peut citer à titre d'exemple d'un tel polyélectrolyte anionique un copolymère obtenu à partir d'un mélange de 30 % en moles d'acide acrylique et de 70 % en moles d'acrylamide.

Les polymères hydrosolubles utilisés dans la présente invention ont une masse moléculaire moyenne en poids supérieure à 1 000 000, de préférence comprise entre 1 000 000 et 50 000 000. Cette masse moléculaire moyenne en poids est déterminée par la méthode RSV *(Reduced Specific Viscosity)* telle que définie dans « Principles of Polymer Chemistry », Cornell University Press, Ithaca, NY, 1953, Chapitre VII intitulé « Determination of Molecular Weight », pages 266 - 316.

La concentration du polymère hydrosoluble de haut poids moléculaire dans la dispersion du compartiment (b1) ou dans la solution du compartiment (b2) est de préférence comprise entre 0,01 et 20 % en poids, rapportée au poids total de la dispersion ou solution.

L'agent alcalin utilisé dans la présente invention pour le défrisage alcalin des fibres kératiniques est une composition aqueuse contenant, à l'état dissous, au moins un hydroxyde minéral ou organique, choisi de préférence parmi les hydroxydes d'un métal alcalin, les hydroxydes d'un métal alcalino-terreux, les hydroxydes d'un métal de transition, les hydroxydes des métaux des groupes III, IV, V et VI, les hydroxydes des lanthanides ou des actinides, les hydroxydes d'ammonium et l'hydroxyde de guanidinium.

L'hydroxyde peut être formé *in situ* comme par exemple l'hydroxyde de guanidine par réaction d'hydroxyde de calcium et de carbonate de guanidine.

Cet agent alcalin doit avoir une concentration suffisante pour permettre la déformation permanente des fibres kératiniques traitées. Une telle déformation permanente est généralement obtenue avec des compositions alcalines aqueuses ayant un pH compris entre 9 et 13, de préférence entre 10 et 13. Ces compositions peuvent contenir en outre des additifs usuels en cosmétique capillaire et en particulier des corps gras.

La présente invention a également pour objet une composition de défrisage prête à l'emploi contenant à la fois un polymère hydrosoluble ayant une masse moléculaire moyenne en poids supérieure à 10⁶ et un agent alcalin en une concentration suffisante pour obtenir une déformation permanente des fibres kératiniques traitées avec cette composition.

Cette composition peut être préparée par mélange
- d'une composition alcaline et
- d'une composition contenant une dispersion de particules d'un polymère hydrosoluble ayant une masse moléculaire moyenne en poids supérieure à 10⁶ dans une solution aqueuse saline, obtenu par polymérisation radicalaire hétérogène de monomères hydrosolubles avec précipitation du polymère formé,
   ou
- d'une solution aqueuse d'un polymère hydrosoluble ayant une masse moléculaire moyenne en poids supérieure à 10⁶ obtenu par polymérisation radicalaire hétérogène de monomères hydrosolubles avec précipitation du polymère formé,
ces différents ingrédients étant ceux décrits en détail ci-dessus.

Le pH de la composition de défrisage prête à l'emploi est de préférence compris entre 9 et 13, et en particulier entre 10 et 13.

Lorsque la composition de défrisage prête à l'emploi est préparée par mélange d'une composition alcaline et d'une composition contenant une dispersion de particules de polymère hydrosoluble, la dilution du milieu de dispersion salin par l'agent aqueux alcalin a pour effet la diminution de la force ionique et la solubilisation totale ou partielle des particules de polymère hydrosoluble.

La proportion de mélange et la concentration de la dispersion ou de la solution de polymère hydrosoluble sont choisies de préférence de manière à ce que la concentration du polymère hydrosoluble soit comprise entre 0,01 et 10 % en poids, de préférence entre 0,05 et 2 % en poids.

La composition de défrisage prête à l'emploi de la présente invention peut contenir en outre un certain nombre d'autres principes actifs cosmétiques ou adjuvants de formulation communément utilisés dans le domaine des soins capillaires. On peut citer à titre d'exemples de tels additifs les agents tensioactifs anioniques, cationiques, non-ioniques ou amphotères, parfums, agents chélatants, agents conservateurs, protéines, silicones, polymères épaississants, huiles végétales, synthétiques ou minérales, cires, agents tampon, filtres UV et colorants capillaires.

La composition prête à l'emploi peut se présenter sous n'importe quelle forme permettant une application et un étalement aisés sur les cheveux. On peut citer à titre d'exemples les émulsions, suspensions, solutions, gels, crèmes et pâtes.

La présente invention a également pour objet des procédés de défrisage des cheveux utilisant séparément la composition alcaline de défrisage et le polymère hydrosoluble de haut poids moléculaire, ou bien la composition de défrisage prête à l'emploi, décrits ci-dessus. Comme indiqué ci-dessus, l'effet de protection des fibres kératiniques par le polymère hydrosoluble de haut poids moléculaire est obtenu aussi bien en cas d'application simultanée de la composition alcaline et du polymère hydrosoluble (procédé en un temps) qu'en cas d'application successive (procédé en deux temps).

Le procédé de défrisage en un temps de la présente invention comprend
(A) l'application d'une composition de défrisage prête à l'emploi telle que décrite ci-dessus sur des cheveux soumis à une contrainte mécanique,
(B) un temps de pose suffisant pour obtenir une déformation permanente des cheveux, et
(C) le rinçage des cheveux.

L'étape (C) peut être suivie d'un shampooing neutralisant.

Le procédé de défrisage en deux temps de la présente invention comprend
(A) l'application, sur les cheveux, d'une composition contenant une dispersion de particules d'un polymère hydrosoluble ayant une masse moléculaire moyenne en poids supérieure à 10⁶ dans une solution aqueuse saline, obtenu par polymérisation radicalaire hétérogène de monomères hydrosolubles avec précipitation du polymère formé, ou d'une composition contenant une solution aqueuse d'un polymère hydrosoluble ayant une masse moléculaire moyenne en poids supérieure à 10⁶ obtenu par polymérisation radicalaire hétérogène de monomères hydrosolubles avec précipitation du polymère formé, suivie éventuellement d'un temps de pose,
(B) éventuellement un rinçage des cheveux,
(C) l'application d'une composition alcaline sur les cheveux soumis à une contrainte mécanique,
(D) un temps de pose suffisant pour obtenir une déformation permanente des cheveux, et
(E) le rinçage des cheveux.

L'étape (E) peut être suivie d'un shampooing neutralisant.

L'ordre d'application du polymère sous forme de dispersion ou de solution (étape A) et de la composition alcaline (étape C) peut être inversé, c'est-à-dire l'application de la composition alcaline peut précéder l'application du polymère hydrosoluble.

Une partie ou l'ensemble des étapes de ces procédés peuvent être effectuées avec application de chaleur.

Le temps de pose suffisant pour obtenir une déformation permanente des cheveux est généralement compris entre 1 et 45 minutes, de préférence entre 1 et 30 minutes.

La présente invention est illustrée à l'aide de l'exemple suivant.

### Exemple 1

### Procédé de défrisage alcalin - Mise en évidence de l'effet protecteur des polymères hydrosolubles de haut poids moléculaire

On applique sur des mèches de cheveux naturels les compositions alcalines de défrisage prête à l'emploi A et B suivantes (pH = 12) à raison de 0,4 g par g de cheveux :

| | Composition A (selon l'invention) | Composition B (comparative) |
|---|---|---|
| NaOH | 1,99 g | 1,99 g |
| Polymère hydrosoluble* | 6,65 g | - |
| Eau | q.s.p. 100 g | q.s.p. 100 g |

| | | |
|---|---|---|
| * Ultimer TX 11415, ONDEO | | |

Après un temps de pose de 20 minutes à température ambiante, les mèches sont rincées, essorées puis séchées.

La dégradation chimique des mèches est évaluée par mesure de la porosité des fibres kératiniques avant et après traitement alcalin.

La porosité des fibres kératiniques se mesure par la fixation à 37 °C, en 2 minutes, de la 2-nitroparaphénylènediamine à 0,25 % dans un mélange éthanol/tampon (rapport volumique 10/90) à pH 10.

Les résultats obtenus sont résumés dans le tableau suivant :

| | Porosité |
|---|---|
| Mèches non traitées | 14 |
| Mèches traitées avec la solution A | 19 |
| Mèches traitées avec la solution B | 37 |

La porosité des mèches traitées par application simultanée d'une composition alcaline et d'un polymère hydrosoluble de haut poids moléculaire (Composition A) est très proche de celle des mèches avant traitement, alors que le traitement alcalin en l'absence de polymère hydrosoluble (Composition B) entraîne une forte augmentation de la porosité, signe d'une dégradation chimique importante des fibres kératiniques.

Les mèches de cheveux traitées avec la composition B présentent en outre des propriétés cosmétiques moins satisfaisantes tels qu'un toucher plus rêche que celui des mèches traitées avec la Composition A.

## Revendications

1. Agent de défrisage alcalin multi-composants comportant,
• en tant que premier composant, (a) une composition alcaline de défrisage, et,
• en tant que deuxième composant, (b1) une composition contenant une dispersion de particules d'un polymère hydrosoluble ayant une masse moléculaire moyenne en poids supérieure à 10⁶ dans une solution aqueuse saline, obtenu par polymérisation radicalaire hétérogène de monomères hydrosolubles avec précipitation du polymère formé ou (b2) une solution aqueuse d'un polymère hydrosoluble ayant une masse moléculaire moyenne en poids supérieure à 10⁶, obtenu par polymérisation radicalaire hétérogène de monomères hydrosolubles avec précipitation du polymère formé, les deux composants (a) et (b1) ou (a) et (b2) étant destinés à être appliqués simultanément ou successivement sur les fibres kératiniques à défriser.

2. Agent de défrisage alcalin multi-composants selon la revendication 1, **caractérisé par le fait qu'**il se présente sous forme d'un kit multi-compartiments pour le défrisage alcalin des fibres kératiniques, avec au moins un premier compartiment contenant le premier composant (a) et au moins un deuxième compartiment contenant le deuxième composant (b1) ou (b2).

3. Agent de défrisage alcalin multi-composants selon la revendication 1 ou 2, **caractérisé par le fait que** le polymère hydrosoluble est un polyélectrolyte.

4. Agent de défrisage alcalin multi-composants selon l'une quelconque des revendications précédentes, **caractérisé par le fait que** le ou les monomères hydrosolubles sont choisis parmi les monomères cationiques, anioniques ou non-ioniques comportant au moins une double liaison éthylénique ou un mélange de ceux-ci.

5. Agent de défrisage alcalin multi-composants selon la revendication 4, **caractérisé par le fait que** les monomères anioniques sont choisis parmi l'acide acrylique, l'acide méthacrylique, l'acide acrylamido-2-méthylpropanesulfonique et l'acide itaconique.

6. Agent de défrisage alcalin multi-composants selon la revendication 4, **caractérisé par le fait que** les monomères non-ioniques sont choisis parmi l'acrylamide, le méthacrylamide, le N-vinylformamide, le N-vinylacétonamide, l'acrylate d'hydroxypropyle et le méthacrylate d'hydroxypropyle.

7. Agent de défrisage alcalin multi-composants selon la revendication 4, **caractérisé par le fait que** les monomères cationiques sont choisis parmi les sels de di(alkyle en C₁₋₄)-diallylammonium et les composés de formule (I) 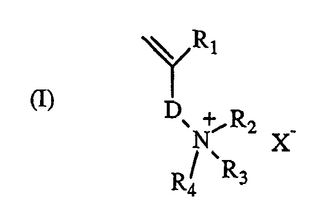 dans laquelle
R₁ représente un atome d'hydrogène ou un groupe méthyle,
R₂ et R₃, identiques ou différents, représentent chacun un atome d'hydrogène ou un groupe alkyle en C₁₋₄ linéaire ou ramifié,
R₄ représente un atome d'hydrogène, un groupe alkyle en C₁₋₄ linéaire ou ramifié ou un groupe aryle,
D représente le motif suivant 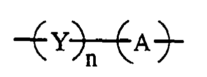 dans lequel Y représente une fonction amide, ester, uréthane ou urée,
A représente un groupe alkyène en C₁₋₁₀ linéaire, ramifié ou cyclique, pouvant être substitué ou interrompu par un cycle aromatique ou hétéroaromatique divalent, ou pouvant être interrompu par un hétéroatome choisi parmi O, N, S et P, et pouvant comporter une fonction cétone, amide, ester, uréthane ou urée,
n vaut 0 ou 1, et
X⁻ représente un contre-ion anionique tel qu'un ion halogénure ou sulfate.

8. Agent de défrisage alcalin multi-composants selon l'une quelconque des revendications précédentes, **caractérisé par le fait que** le polymère hydrosoluble est polymérisé à partir d'au moins un monomère cationique de formule (I).

9. Agent de défrisage alcalin multi-composants selon la revendication 8, **caractérisé par le fait que** le polymère hydrosoluble est polymérisé à partir d'un mélange de monomères constitué de 0 à 30 % en moles d'acide acrylique, de 0 à 95,5 % en moles d'acrylamide et de 0,5 à 10 % en moles d'au moins un monomère cationique de formule (I).

10. Agent de défrisage alcalin multi-composants selon la revendication 8, **caractérisé par le fait que** le polymère hydrosoluble est polymérisé à partir d'un mélange de monomères constitué d'acide acrylique et d'un monomère cationique de formule (I), le nombre de moles du monomère cationique de formule (I) étant supérieur au nombre de moles d'acide acrylique.

11. Agent de défrisage alcalin multi-composants selon la revendication 8, **caractérisé par le fait que** le polymère hydrosoluble est polymérisé à partir d'un mélange de monomères constitué de 10 % en moles de chlorure d'acryloyloxyéthyldiméthylbenzyl-ammonium et de 90 % en moles d'acrylamide.

12. Agent de défrisage alcalin multi-composants selon la revendication 8, **caractérisé par le fait que** le polymère hydrosoluble est polymérisé à partir d'un mélange de monomères constitué de 30 % en moles de chlorure d'acryloyloxytriméthylammonium, de 50 % en moles de chlorure d'acryloyloxyéthyldiméthybenzylammonium et de 20 % en moles d'acrylamide.

13. Agent de défrisage alcalin multi-composants selon la revendication 8, **caractérisé par le fait que** le polymère hydrosoluble est polymérisé à partir de 10 % en moles de chlorure d'acryloyloxyéthyltriméthylammonium et de 90 % en moles d'acrylamide.

14. Agent de défrisage alcalin multi-composants selon la revendication 8, **caractérisé par le fait que** le polymère hydrosoluble est polymérisé à partir de 30 % en moles de chlorure de diallyldiméthylammonium et de 70 % en moles d'acrylamide.

15. Agent de défrisage alcalin multi-composants l'une des revendications 1 à 6, **caractérisé par le fait que** le polymère hydrosoluble est un polymère anionique polymérisé à partir de 30 % en moles d'acide acrylique et de 70 % en moles d'acrylamide.

16. Agent de défrisage alcalin multi-composants selon l'une quelconque des revendications précédentes, **caractérisé par le fait que** la concentration des particules de polymère hydrosoluble en dispersion dans une solution aqueuse saline dans le compartiment (b1) est comprise entre 0,01 et 20 % en poids.

17. Agent de défrisage alcalin multi-composants selon l'une quelconque des revendications 1 à 16, **caractérisé par le fait que** la concentration du polymère hydrosoluble en solution dans le compartiment (b2) est comprise entre 0,01 et 20 % en poids.

18. Agent de défrisage alcalin multi-composants selon l'une quelconque des revendications précédentes, **caractérisé par le fait que** la solution aqueuse saline dans le compartiment (b1) ou (b2) contient au moins un sel anionique divalent.

19. Agent de défrisage alcalin multi-composants selon la revendication 18, **caractérisé par le fait que** le sel anionique divalent est choisi parmi le sulfate d'ammonium, l'hydrogénosulfate d'ammonium, le sulfate de sodium, l'hydrogénosulfate de sodium, le sulfate de magnésium, l'hydrogénosulfate de magnésium, le sulfate d'aluminium, l'hydrogénosulfate d'aluminium.

20. Agent de défrisage alcalin multi-composants selon l'une quelconque des revendications précédentes, **caractérisé par le fait que** la composition alcaline a un pH compris entre 9 et 13, de préférence entre 10 et 13.

21. Agent de défrisage alcalin multi-composants selon l'une quelconque des revendications précédentes, **caractérisé par le fait que** la composition alcaline est une solution aqueuse d'au moins un hydroxyde minéral ou organique choisi parmi les hydroxydes d'un métal alcalin, les hydroxydes d'un métal alcalino-terreux, les hydroxydes d'un métal de transition, les hydroxydes des métaux des groupes III, IV, V et VI, les hydroxydes des lanthanides ou des actinides, les hydroxydes d'ammonium et l'hydroxyde de guanidinium.

22. Composition de défrisage prête à l'emploi contenant à la fois un polymère hydrosoluble ayant une masse moléculaire moyenne en poids supérieure à 10⁶ et un agent alcalin en une concentration suffisante pour obtenir une déformation permanente des fibres kératiniques traitées avec cette composition

23. Composition de défrisage prête à l'emploi selon la revendication 22, **caractérisée par le fait qu'**elle est obtenue par mélange
• d'une composition alcaline et
• d'une composition contenant une dispersion de particules d'un polymère hydrosoluble ayant une masse moléculaire moyenne en poids supérieure à 10⁶ dans une solution aqueuse saline, obtenue par polymérisation radicalaire hétérogène de monomères hydrosolubles avec précipitation du polymère formé,
ou
• d'une solution aqueuse d'un polymère hydrosoluble ayant une masse moléculaire moyenne en poids supérieure à 10⁶, obtenu par polymérisation radicalaire hétérogène de monomères hydrosolubles avec précipitation du polymère formé, tels que définis dans les revendications précédentes.

24. Composition de défrisage prête à l'emploi selon la revendication 22 ou 23 **caractérisée par le fait qu'**elle a un pH compris entre 9 et 13, de préférence compris entre 10 et 13.

25. Composition de défrisage prête à l'emploi selon l'une quelconque des revendications 22 à 24, **caractérisée par le fait que** la concentration en polymère hydrosoluble ayant une masse moléculaire moyenne en poids supérieure à 10⁶ est comprise entre 0,01 et 10 % en poids, de préférence entre 0,05 et 2 % en poids.

26. Procédé de défrisage des cheveux comprenant
(A) l'application d'une composition de défrisage prête à l'emploi selon l'une quelconque des revendications 22 à 25 sur des cheveux soumis à une contrainte mécanique,
(B) un temps de pose suffisant pour obtenir une déformation permanente des cheveux, et
(C) le rinçage des cheveux.

27. Procédé de défrisage des cheveux comprenant
(A) l'application, sur les cheveux, d'une composition contenant une dispersion de particules d'un polymère hydrosoluble ayant une masse moléculaire moyenne en poids supérieure à 10⁶ dans une solution aqueuse saline, obtenu par polymérisation radicalaire hétérogène de monomères hydrosolubles avec précipitation du polymère formé, ou d'une composition contenant une solution aqueuse d'un polymère hydrosoluble ayant une masse moléculaire moyenne en poids supérieure à 10⁶ obtenu par polymérisation radicalaire hétérogène de monomères hydrosolubles avec précipitation du polymère formé, suivie éventuellement d'un temps de pose,
(B) éventuellement un rinçage des cheveux,
(C) l'application d'une composition alcaline sur les cheveux soumis à une contrainte mécanique,
(D) un temps de pose suffisant pour obtenir une déformation permanente des cheveux, et
(E) le rinçage des cheveux.

28. Procédé de défrisage des cheveux selon la revendication 27, **caractérisé par le fait que** l'ordre des étapes (A) et (C) est inversé.

## Claims

1. Multi-component alkaline hair-relaxing agent comprising:
• as first component, (a) an alkaline hair-relaxing composition, and
• as second component, (b1) a composition containing a dispersion of particles of a water-soluble polymer having a weight-average molecular mass of greater than 10⁶ in a saline aqueous solution, obtained by heterogeneous free-radical polymerization of water-soluble monomers with precipitation of the formed polymer, or (b2) an aqueous solution of a water-soluble polymer with a weight-average molecular mass of,greater than 10⁶, obtained by heterogeneous free-radical polymerization of water-soluble monomers with precipitation of the formed polymer,
the two components (a) and (b1) or (a) and (b2) being intended to be applied simultaneously or successively to the keratin fibres to be relaxed.

2. Multi-component alkaline hair-relaxing agent according to Claim 1, **characterized in that** it is in the form of a multi-compartment kit for the alkaline relaxing of keratin fibres, with at least one first compartment containing the first component (a) and at least one second compartment containing the second component (b1) or (b2).

3. Multi-component alkaline hair-relaxing agent according to Claim 1 or 2, **characterized in that** the water-soluble polymer is a polyelectrolyte.

4. Multi-component alkaline hair-relaxing agent according to any one of the preceding claims, **characterized in that** the water-soluble monomer(s) is (are) chosen from cationic, anionic and nonionic monomers comprising at least one ethylenic double bond, or a mixture thereof.

5. Multi-component alkaline hair-relaxing agent according to Claim 4, **characterized in that** the anionic monomers are chosen from acrylic acid, methacrylic acid, acrylamido-2-methylpropanesulfonic acid and itaconic acid.

6. Multi-component alkaline hair-relaxing agent according to Claim 4, **characterized in that** the nonionic monomers are chosen from acrylamide, methacrylamide, N-vinylformamide, N-vinylacetonamide, hydroxypropyl acrylate and hydroxypropyl methacrylate.

7. Multi-component hair-relaxing agent according to Claim 4, **characterized in that** the cationic monomers are chosen from di(C₁₋₄ alkyl)diallylammonium salts and the compounds of formula (I) 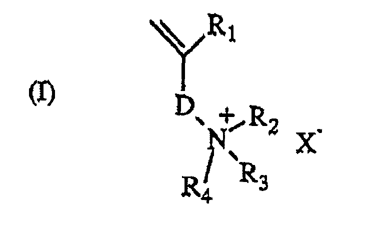 in which
R₁ represents a hydrogen atom or a methyl group,
R₂ and R₃, which may be identical or different, each represent a hydrogen atom or a linear or branched C₁₋₄ alkyl group,
R₄ represents a hydrogen atom, a linear or branched C₁₋₄ alkyl group or an aryl group,
D represents the following unit 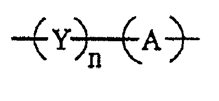 in which Y represents an amide, ester, urethane or urea function,
A represents a linear, branched or cyclic C₁₋₁₀ alkylene group, which may be substituted or interrupted with a divalent aromatic or heteroaromatic ring, or which may be interrupted with a hetero atom chosen from O, N, S and P, and which may comprise a ketone, amide, ester, urethane or urea function,
n is 0 or 1, and
X⁻ represents an anionic counterion such as a chloride or sulfate ion.

8. Multi-component alkaline hair-relaxing agent according to any one of the preceding claims, **characterized in that** the water-soluble polymer is polymerized from at least one cationic monomer of formula (I).

9. Multi-component alkaline hair-relaxing agent according to Claim 8, **characterized in that** the water-soluble polymer is polymerized from a monomer blend consisting of from 0 to 30 mol% of acrylic acid, from 0 to 95.5 mol% of acrylamide and from 0.5 mol% to 10 mol% of at least one cationic monomer of formula (I) .

10. Multi-component alkaline hair-relaxing agent according to Claim 8, **characterized in that** the water-soluble polymer is polymerized from a monomer blend consisting of acrylic acid and of a cationic monomer of formula (I), the number of moles of the cationic monomer of formula (I) being greater than the number of moles of acrylic acid.

11. Multi-component alkaline hair-relaxing agent according to Claim 8, **characterized in that** the water-soluble polymer is polymerized from a monomer blend consisting of 10 mol% of acryloyloxyethyldimethylbenzylammonium chloride and 90 mol% of acrylamide.

12. Multi-component alkaline hair-relaxing agent according to Claim 8, **characterized in that** the water-soluble polymer is polymerized from a monomer blend consisting of 30 mol% of acryloyloxytrimethylammonium chloride, 50 mol% of acryloyloxyethyldimethylbenzylammonium chloride and 20 mol% of acrylamide.

13. Multi-component alkaline hair-relaxing agent according to Claim 8, **characterized in that** the water-soluble polymer is polymerized from 10 mol% of acryloyloxyethyltrimethylammonium chloride and 90 mol% of acrylamide.

14. Multi-component alkaline hair-relaxing agent according to Claim 8, **characterized in that** the water-soluble polymer is polymerized from 30 mol% of diallyldimethylammonium chloride and 70 mol% of acrylamide.

15. Multi-component alkaline hair-relaxing agent according to one of Claims 1 to 6, **characterized in that** the water-soluble polymer is an anionic polymer polymerized from 30 mol% of acrylic acid and 70 mol% of acrylamide.

16. Multi-component alkaline hair-relaxing agent according to any one of the preceding claims,
**characterized in that** the concentration of water-soluble polymer particles in dispersion in a saline aqueous solution in compartment (b1) is between 0.01% and 20% by weight.

17. Multi-component alkaline hair-relaxing agent according to any one of Claims 1 to 16, **characterized in that** the concentration of the water-soluble polymer in solution in compartment (b2) is between 0.01% and 20% by weight.

18. Multi-component alkaline hair-relaxing agent according to any one of the preceding claims, **characterized in that** the saline aqueous solution in compartment (b1) or (b2) contains at least one divalent anionic salt.

19. Multi-component alkaline hair-relaxing agent according to Claim 18, **characterized in that** the divalent anionic salt is chosen from ammonium sulfate, ammonium hydrogen sulfate, sodium sulfate, sodium hydrogen sulfate, magnesium sulfate, magnesium hydrogen sulfate, aluminium sulfate and aluminium hydrogen sulfate.

20. Multi-component alkaline hair-relaxing agent according to any one of the preceding claims, **characterized in that** the alkaline composition has a pH of between 9 and 13 and preferably between 10 and 13.

21. Multi-component alkaline hair-relaxing agent according to any one of the preceding claims, **characterized in that** the alkaline composition is an aqueous solution of at least one mineral or organic hydroxide chosen from hydroxides of an alkali metal, hydroxides of an alkaline-earth metal, hydroxides of a transition metal, hydroxides of metals from groups III, IV, V and VI, lanthanide or actinide hydroxides, ammonium hydroxides and guanidinium hydroxide.

22. Ready-to-use hair-relaxing composition containing both a water-soluble polymer with a weight-average molecular mass of greater than 10⁶ and an alkaline agent in a concentration that is sufficient to permanently reshape keratin fibres treated with this composition.

23. Ready-to-use hair-relaxing composition according to Claim 22, **characterized in that** it is obtained by mixing:
• an alkaline composition and
• a composition containing a dispersion of particles of a water-soluble polymer with a weight-average molecular mass of greater than 10⁶ in a saline aqueous solution, obtained by heterogeneous free-radical polymerization of water-soluble monomers with precipitation of the formed polymer, or
• an aqueous solution of a water-soluble polymer with a weight-average molecular mass of greater than 10⁶ obtained by heterogeneous free-radical polymerization of water-soluble monomers with precipitation of the formed polymer,
as defined in the preceding claims.

24. Ready-to-use hair-relaxing composition according to Claim 22 or 23, **characterized in that** it has a pH of between 9 and 13 and preferably of between 10 and 13.

25. Ready-to-use hair-relaxing composition according to any one of Claims 22 to 24, **characterized in that** the concentration of water-soluble polymer with a weight-average molecular mass of greater than 10⁶ is between 0.01% and 10% by weight and preferably between 0.05% and 2% by weight.

26. Hair-relaxing process comprising:
(A) the application of a ready-to-use hair-relaxing composition according to any one of Claims 22 to 25 to hair subjected to a mechanical constraint,
(B) a leave-in time that is sufficient to obtain permanent reshaping of the hair, and
(C) rinsing of the hair.

27. Hair-relaxing process comprising:
(A) the application, to the hair, of a composition containing a dispersion of particles of a water-soluble polymer with a weight-average molecular mass of greater than 10⁶ in a saline aqueous solution, obtained by heterogeneous free-radical polymerization of water-soluble monomers with precipitation of the formed polymer, or of a composition containing an aqueous solution of a water-soluble polymer with a weight-average molecular mass of greater than 10⁶ obtained by heterogeneous free-radical polymerization of water-soluble monomers with precipitation of the formed polymer, optionally followed by a leave-in time,
(B) optionally, rinsing of the hair,
(C) the application of an alkaline composition to the hair subjected to a mechanical constraint,
(D) a leave-in time that is sufficient to obtain permanent reshaping of the hair, and
(E) rinsing of the hair.

28. Hair-relaxing process according to Claim 27, **characterized in that** the order of steps (A) and (C) is reversed.

## Patentansprüche

1. Alkalisches Haarglättungsmittel mit mehreren Komponenten, umfassend:
. als erste Komponente (a) eine alkalische Zusammensetzung für die Haarglättung,
. als zweite Komponente (b1) eine Zusammensetzung, die in einer wässrigen Salzlösung eine Dispersion von Partikeln eines wasserlöslichen Polymers mit einer gewichtsmittleren Molmasse über 10⁶ enthält, das durch heterogene radikalische Polymerisation von wasserlöslichen Monomeren unter Präzipitation des gebildeten Polymers hergestellt wird, oder (b2) eine wässrige Lösung eines wasserlöslichen Polymers mit einer gewichtsmittleren Molmasse über 10⁶, das durch heterogene radikalische Polymerisation von wasserlöslichen Monomeren unter Präzipitation des gebildeten Polymers hergestellt wird,
wobei die beiden Komponenten (a) und (b1) oder (a) und (b2) dazu vorgesehen sind, gleichzeitig oder nacheinander auf die zu entkräuselnden Keratinfasern aufgetragen zu werden.

2. Alkalisches Haarglättungsmittel mit mehreren Komponenten nach Anspruch 1, **dadurch gekennzeichnet, dass** es in der Form eines Kits für die alkalische Haarglättung von Keratinfasern mit mehreren Abteilungen vorliegt, mit mindestens einer ersten Abteilung, die die erste Komponente (a) enthält, und mindestens einer zweiten Abteilung, die die zweite Komponente (b1) oder (b2) enthält.

3. Alkalisches Haarglättungsmittel mit mehreren Komponenten nach Anspruch 1 oder 2, **dadurch gekennzeichnet, dass** das wasserlösliche Polymer ein Polyelektrolyt ist.

4. Alkalisches Haarglättungsmittel mit mehreren Komponenten nach einem der vorhergehenden Ansprüche, **dadurch gekennzeichnet, dass** das oder die wasserlöslichen Monomere unter den kationischen, anionischen oder nichtionischen Monomeren, die mindestens eine ethylenische Doppelbindung aufweisen, oder deren Gemischen ausgewählt sind.

5. Alkalisches Haarglättungsmittel mit mehreren Komponenten nach Anspruch 4, **dadurch gekennzeichnet, dass** die anionischen Monomere unter Acrylsäure, Methacrylsäure, Acrylamido-2-methylpropansulfonsäure und Itaconsäure ausgewählt sind.

6. Alkalisches Haarglättungsmittel mit mehreren Komponenten nach Anspruch 4, **dadurch gekennzeichnet, dass** die nichtionischen Monomere unter Acrylamid, Methacrylamid, N-Vinylformamid, N-Vinylacetamid, Hydroxypropylacrylat und Hydroxypropylmethacrylat ausgewählt sind.

7. Alkalisches Haarglättungsmittel mit mehreren Komponenten nach Anspruch 4, **dadurch gekennzeichnet, dass** die kationischen Monomere unter den Di(alkyl(C₁₋₄))diallylammoniumsalzen und den Verbindungen der Formel (I) ausgewählt sind: 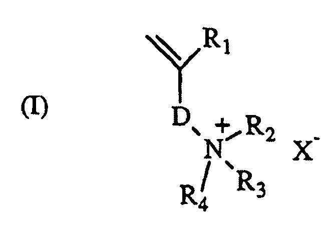 in der Formel:
R₁ bedeutet ein Wasserstoffatom oder eine Methylgruppe,
R₂ und R₃, die gleich oder verschieden sind, bedeuten jeweils ein
Wasserstoffatom oder eine geradkettige oder verzweigte C₁₋₄-Alkylgruppe,
R₄ bedeutet ein Wasserstoffatom, eine geradkettige oder verzweigte C₁₋₄-Alkylgruppe oder eine Arylgruppe,
D ist die folgende Einheit 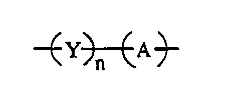
worin Y eine Amidfunktion, Esterfunktion, Urethanfunktion oder Harnstofffunktion bedeutet,
A ist eine geradkettige, verzweigte oder cyclische C₁₋₁₀-Alkylengruppe, die substituiert oder mit einem zweiwertigen aromatischen oder heteroaromatischen Ring unterbrochen sein kann, oder durch ein Heteroatom, das unter O, N, S und P ausgewählt ist, unterbrochen sein kann und eine Ketonfunktion, Amidfunktion, Esterfunktion, Urethanfunktion oder Harnstofffunktion enthalten kann,
n beträgt 0 oder 1, und
X⁻ bedeutet ein anionisches Gegenion, wie ein Halogenid oder Sulfat.

8. Alkalisches Haarglättungsmittel mit mehreren Komponenten nach einem der vorhergehenden Ansprüche, **dadurch gekennzeichnet, dass** das wasserlösliche Polymer ausgehend von mindestens einem kationischen Polymer der Formel (I) polymerisiert wird.

9. Alkalisches Haarglättungsmittel mit mehreren Komponenten nach Anspruch 8, **dadurch gekennzeichnet, dass** das wasserlösliche Polymer ausgehend von einem Monomerengemisch polymerisiert wird, das aus 0 bis 30 Mol-% Acrylsäure, 0 bis 95,5 Mol-% Acrylamid und 0,5 bis 10 Mol-% mindestens eines kationischen Monomers der Formel (I) besteht.

10. Alkalisches Haarglättungsmittel mit mehreren Komponenten nach Anspruch 8, **dadurch gekennzeichnet, dass** das wasserlösliche Polymer ausgehend von einem Monomerengemisch polymerisiert wird, das aus Acrylsäure und einem kationischen Monomer der Formel (I) besteht, wobei die Anzahl der Mol des kationischen Monomers der Formel (I) größer ist als die Anzahl der Mol der Acrylsäure.

11. Alkalisches Haarglättungsmittel mit mehreren Komponenten nach Anspruch 8, **dadurch gekennzeichnet, dass** das wasserlösliche Polymer ausgehend von einem Monomerengemisch polymerisiert wird, das aus 10 Mol-% Acryloyloxyethyldimethylbenzylammoniumchlorid und 90 Mol-% Acrylamid besteht.

12. Alkalisches Haarglättungsmittel mit mehreren Komponenten nach Anspruch 8, **dadurch gekennzeichnet, dass** das wasserlösliche Polymer ausgehend von einem Monomerengemisch polymerisiert wird, das aus 30 Mol-% Acryloyloxytrimethylammoniumchlorid, 50 Mol-% Acryloyloxyethyldimethylbenzylammoniumchlorid und 20 Mol-% Acrylamid besteht.

13. Alkalisches Haarglättungsmittel mit mehreren Komponenten nach Anspruch 8, **dadurch gekennzeichnet, dass** das wasserlösliche Polymer ausgehend von 10 Mol-% Acryloyloxyethyltrimethylammoniumchlorid und 90 Mol-% Acrylamid polymerisiert wird.

14. Alkalisches Haarglättungsmittel mit mehreren Komponenten nach Anspruch 8, **dadurch gekennzeichnet, dass** das wasserlösliche Polymer ausgehend von 30 Mol-% Diallyldimethylammoniumchlorid und 70 Mol-% Acrylamid polymerisiert wird.

15. Alkalisches Haarglättungsmittel mit mehreren Komponenten nach einem der Ansprüche 1 bis 6, **dadurch gekennzeichnet, dass** das wasserlösliche Polymer ein anionisches Polymer ist, das ausgehend von 30 Mol-% Acrylsäure und 70 Mol-% Acrylamid polymerisiert wird.

16. Alkalisches Haarglättungsmittel mit mehreren Komponenten nach einem der vorhergehenden Ansprüche, **dadurch gekennzeichnet, dass** die Konzentration der Partikel des wasserlöslichen Polymers in Dispersion in einer wässrigen Salzlösung in der Komponente (b1) im Bereich von 0,01 bis 20 Gew.-% liegt.

17. Alkalisches Haarglättungsmittel mit mehreren Komponenten nach einem der Ansprüche 1 bis 16, **dadurch gekennzeichnet, dass** die Konzentration des wasserlöslichen Polymers in Lösung in der Komponente (b2) im Bereich von 0,01 bis 20 Gew.-% liegt.

18. Alkalisches Haarglättungsmittel mit mehreren Komponenten nach einem der vorhergehenden Ansprüche, **dadurch gekennzeichnet, dass** die wässrige Salzlösung in der Komponente (b1) oder (b2) mindestens ein Salz eines zweiwertigen Anions enthält.

19. Alkalisches Haarglättungsmittel mit mehreren Komponenten nach Anspruch 18, **dadurch gekennzeichnet, dass** das Salz eines zweiwertigen Anions unter Ammoniumsulfat, Ammoniumhydrogensulfat, Natriumsulfat, Natriumhydrogensulfat, Magnesiumsulfat, Magnesiumhydrogensulfat, Aluminiumsulfat und Aluminiumhydrogensulfat ausgewählt ist.

20. Alkalisches Haarglättungsmittel mit mehreren Komponenten nach einem der vorhergehenden Ansprüche, **dadurch gekennzeichnet, dass** die alkalische Zusammensetzung einen pH-Wert im Bereich von 9 bis 13 und vorzugsweise 10 bis 13 aufweist.

21. Alkalisches Haarglättungsmittel mit mehreren Komponenten nach einem der vorhergehenden Ansprüche, **dadurch gekennzeichnet, dass** die alkalische Zusammensetzung eine wässrige Lösung mindestens eines anorganischen oder organisches Hydroxids ist, das unter den Alkalimetallhydroxiden, Erdalkalimetallhydroxiden, Hydroxiden von Übergangsmetallen, Hydroxiden von Metallen der Gruppen III, IV, V und VI, Hydroxiden von Lanthaniden oder Actinoiden, Hydroxiden von Ammonium und Guanidiniumhydroxid ausgewählt ist.

22. Gebrauchsfertige Zusammensetzung für die Haarglättung, die gleichzeitig ein wasserlösliches Polymer mit einer gewichtsmittleren Molmasse über 10⁶ und einen alkalischen Stoff in einer Konzentration enthält, die ausreichend ist, um eine dauerhafte Verformung der mit dieser Zusammensetzung behandelten Keratinfasern zu bewirken.

23. Gebrauchsfertige Zusammensetzung für die Haarglättung nach Anspruch 22, **dadurch gekennzeichnet, dass** sie erhalten wird durch Mischen
. einer alkalischen Zusammensetzung und
. einer Zusammensetzung, die eine Dispersion von Partikeln eines wasserlöslichen Polymers mit einer gewichtsmittleren Molmasse über 10⁶ in einer wässrigen Salzlösung enthält, das durch heterogene radikalische Polymerisation von wasserlöslichen Monomeren unter Präzipitation des gebildeten Polymers hergestellt wird, oder
. einer wässrigen Lösung eines wasserlöslichen Polymers mit einer gewichtsmittleren Molmasse über 10⁶, das durch heterogene radikalische Polymerisation von wasserlöslichen Monomeren unter Präzipitation des gebildeten Polymers hergestellt wird,
wie sie in einem der vorhergehenden Ansprüche definiert wurden.

24. Gebrauchsfertige Zusammensetzung für die Haarglättung nach Anspruch 22 oder 23, **dadurch gekennzeichnet, dass** sie einen pH-Wert im Bereich von 9 bis 13 und vorzugsweise 10 bis 13 aufweist.

25. Gebrauchsfertige Zusammensetzung für die Haarglättung nach einem der Ansprüche 22 bis 24, **dadurch gekennzeichnet, dass** die Konzentration des wasserlösliches Polymers, das eine gewichtsmittlere Molmasse über 10⁶ aufweist, im Bereich von 0,01 bis 10 Gew.-% und vorzugsweise 0,05 bis 2 Gew.-% liegt.

26. Verfahren zum Glätten von Haaren, das umfasst:
(A) das Auftragen einer gebrauchsfertigen Zusammensetzung für die Haarglättung nach einem der Ansprüche 22 bis 25 auf Haare, die unter mechanische Spannung gesetzt wurden,
(B) eine Einwirkzeit, die ausreichend ist, um eine dauerhafte Verformung der Haare zu erzielen, und
(C) das Spülen der Haare.

27. Verfahren zum Glätten von Haaren, das umfasst:
(A) das Auftragen einer Zusammensetzung, die in einer wässrigen Salzlösung eine Dispersion von Partikeln eines wasserlöslichen Polymers mit einer gewichtsmittleren Molmasse über 10⁶ enthält, das durch heterogene radikalische Polymerisation von wasserlöslichen Monomeren unter Präzipitation des gebildeten Polymers hergestellt wird, oder einer Zusammensetzung, die eine wässrige Lösung eines wasserlöslichen Polymers mit einer gewichtsmittleren Molmasse über 10⁶ enthält, das durch heterogene radikalische Polymerisation von wasserlöslichen Monomeren unter Präzipitation des gebildeten Polymers hergestellt wird, auf die Haare, wonach gegebenenfalls eine Einwirkzeit abgewartet wird,
(B) gegebenenfalls das Spülen der Haare,
(C) das Auftragen einer alkalischen Zusammensetzung auf die Haare, die zuvor unter mechanische Spannung gesetzt wurden,
(D) eine Einwirkzeit, die ausreichend ist, um eine dauerhafte Verformung der Haare zu erzielen, und
(E) das Spülen der Haare.

28. Verfahren zum Glätten von Haaren nach Anspruch 27, **dadurch gekennzeichnet, dass** die Reihenfolge der Schritte (A) und (C) umgekehrt ist.
